# EUROPEAN PATENT APPLICATION

(11) **EP 3 646 777 A1**
(43) Date of publication of application: **06.05.2020**
(21) Application number: 18823660.8
(22) Date of filing: 28.06.2018
(51) Int. Cl.: A61B 5/00, A61B 5/0444, A61B 5/0452

(54) **MATERNAL AND FETAL MONITORING DEVICE AND MONITORING DEVICE DISPLAY DEVICE**

(30) Priority: 29.06.2017 JP 2017127544
(71) Applicant: Atom Medical Corporation, Bunkyo-ku Tokyo 113-0033 (JP)
(72) Inventor: SUDO, Kazuhiko, Saitama City Saitama 338-0835 (JP); ODAGIRI, Naoko, Saitama City Saitama 338-0835 (JP); OOWADA, Kazunari, Saitama City Saitama 338-0835 (JP); KUBO, Masayuki, Saitama City Saitama 338-0835 (JP); SONE, Yoshikatsu, Saitama City Saitama 338-0835 (JP); MATSUBARA, Ichiro, Tokyo 113-0033 (JP)
(74) Representative: DREISS Patentanwälte PartG mbB
(86) International application number: PCT/JP2018/024703
(87) International publication number: WO 2019/004394

(57) **Abstract**

There are provided a maternal and fetal monitoring device and a display device for monitoring device including fetal heart rate acquisition means configured to acquire a fetal heart rate, labor pain intensity acquisition means configured to acquire a maternal labor pain intensity, fetal bioelectric signal acquisition means configured to acquire a fetal bioelectric signal, and display means capable of simultaneously displaying a cardiotocogram that displays the fetal heart rate and the labor pain intensity side by side on the same time axis over time as a graph and a fetal bioelectric signal diagram displaying the fetal bioelectric signal, and optimizing and displaying, together with the cardiotocogram, the fetal bioelectric signal diagram and the like closely related to these pieces of information.

## Description

### Technical Field

The present invention relates to a maternal and fetal monitoring device that monitor and record fetal heart rate, uterine contraction pressure, and the like, and a display device for monitoring device.

Priority is claimed on Japanese Patent Application No. 2017-127544, filed on June 29, 2017, the content of which is incorporated herein by reference.

### Background Art

It is known that regular uterine contractions occur during a delivery, and changes in a fetal heart rate in response to the uterine contractions are observed to presume a fetal situation. Further, a cardiotocogram (CTG) is used in which the fetal heart rate and a uterine contraction pressure (labor pain intensity) are recorded with time in order to grasp the fetal situation.

For example, Patent Literature 1 describes a delivery monitoring device that records a fetal heart rate curve indicating a change in the fetal heart rate(FHR) over time and a maternal labor pain curve indicating a change in the labor pain intensity (uterine contraction pressure) over time on a recording sheet. A display unit in this delivery monitoring device is provided with a display unit relating to transient bradycardia (early, delayed, variant, and prolonged), a display unit relating to heart rate baseline fine variation (disappearance, decrease, moderate, and increase), and a warning sign are provided together with a fetal heart rate display unit in which the fetal heart rate is digitally displayed (numerical display), a labor pain intensity display unit in which the labor pain intensity is digitally displayed (numerical display), and a fetal heart rate baseline display unit in which a fetal heart rate baseline is digitally displayed (numerical display). As described above, Patent Literature 1 describes that main information such as the fetal heart rate baseline and a warning such as urgency are displayed on the display unit of the delivery monitoring device. Therefore, a high level of interpretation work for determining a health state of a fetus is unnecessary and a burden on a doctor is reduced.

As methods of detecting the fetal heart rate in such a delivery monitoring device in the related art, there are an internal measurement method of directly attaching electrocardiographic electrodes to the fetus after rupture (during delivery) to directly detect a fetal electrocardiogram and an external measurement method (ultrasonic Doppler method) of indirectly detecting a movement of a fetal heart by an ultrasonic transducer attached on the maternal abdominal wall. Of the methods, the internal measurement method cannot be used before the rupture since the electrocardiogram electrode is directly attached to the fetus.

In the ultrasonic Doppler method (external measurement method) that can be used before the rupture, an ultrasonic wave is transmitted from the ultrasonic transducer attached to the maternal abdominal wall toward the heart of the fetus in the maternal body and the ultrasonic transducer receives a reflected wave reflected by the heart to measure the heart rate of the fetus based on the received signal. The received signal obtained by such an ultrasonic Doppler method includes a signal of a heart valve, myocardium, or the like derived from the movement of the heart and a signal derived from a maternal heartbeat, a movement of the uterine contraction or the like. Therefore, for example, the fetal heart rate is calculated by storing the received signal at constant time intervals and subjecting to autocorrelation processing to detect periodicity of the heart rate as described in Patent Literature 2.

### Citation List

### Patent Literature

Patent Literature 1: JP-A-2006-223335
Patent Literature 2: JP-A-10-28686
Patent Literature 3: Japanese Patent No. 4590554

### SUMMARY OF INVENTION

### Technical Problem

However, in the conventional delivery monitoring device using such an ultrasonic Doppler method, since it is necessary to subject to the autocorrelation processing on the received signal stored at the constant time intervals, it is impossible to acquire the fetal heart rate in real time. For this reason, arrhythmia and the variation in the heart rate within a sampling time of the fetus cannot be observed, and a heart rate different from an actual heart rate may be recorded as a measurement value. In the calculation method of the fetal heart rate using the autocorrelation processing, short term variability (STV) or long term variability (LTV) cannot be measured accurately. Therefore, a discrepancy may occur between an actual clinical symptom and a measurement record and there is a risk of misunderstanding of diagnosis in a medical field.

For example, Patent Literature 3 discloses an electrocardiogram signal processing method and an electrocardiogram signal processing device for extracting a fetal electrocardiogram signal (a signal corresponding to a fetal bioelectric signal) included in a biopotential signal (abdominal electrocardiogram signal) detected from electrodes attached to a maternal body. As described in Patent Literature 3, the usefulness of the fetal bioelectric signal is widely recognized, and a method of extracting the fetal bioelectric signal not only during the delivery but also by a non-invasive method is considered.

When the fetal bioelectric signal can be extracted accurately, it is considered that it is possible to perform accurate heart rate detection between R-R' (R wave intervals), it is possible to accurately grasp the variation in the fetal heart rate by beat-to-beat (every heartbeat), and further it is easy to determine the situation by comparing the fetal heart rate curve indicating the change in the fetal heart rate over time, the maternal labor pain curve indicating the change in the labor pain intensity (uterine contraction pressure) over time, and the fetal bioelectric signal diagram. For this reason, there is a need for a monitoring device that is optimized for smoothly using the fetal bioelectric signal in determining the fetal situation by displaying the fetal bioelectric signal together with the fetal heart rate curve and the maternal labor pain curve.

The present invention has been made in view of such circumstances, and an object is to provide a maternal and fetal monitoring device and a monitoring device display device capable of easily and correctly determining a maternal and fetal situation by optimizing and displaying, together with a cardiotocogram that displays a fetal heart rate (fetal heart rate diagram) and a maternal labor pain intensity (labor pain intensity diagram) side by side over time, information such as a fetal bioelectric signal closely related to these pieces of information.

### Solution to Problem

A maternal and fetal monitoring device of the invention includes fetal heart rate acquisition means configured to acquire fetal heart rate, labor pain intensity acquisition means configured to acquire a maternal labor pain intensity, fetal bioelectric signal acquisition means configured to acquire a fetal bioelectric signal, and display means capable of simultaneously displaying a cardiotocogram that displays the fetal heart rate and the labor pain intensity side by side on the same time axis over time as a graph and a fetal bioelectric signal diagram that displays the fetal bioelectric signal.

The fetal bioelectric signal diagram is displayed together with the cardiotocogram on the same time axis. Therefore, it becomes easy to find the correlation among the fetal heart rate, the labor pain intensity, and the fetal bioelectric signal, and it is possible to resolve the misunderstanding in the diagnosis based on only the cardiotocogram. Further, it is possible to shorten an operation time by simultaneously displaying the cardiotocogram and the fetal bioelectric signal diagram (fetal bioelectric signal waveform).

The maternal and fetal monitoring device of the invention may further include a designated period input unit capable of designating a predetermined period on the cardiotocogram. The display means may be able to display the fetal bioelectric signal in the predetermined period on the fetal bioelectric signal diagram when the predetermined period is designated by the designated period input unit.

It is possible to check the variation in the fetal bioelectric signal in the predetermined period in the cardiotocogram in addition to the cardiotocogram and thus to easily find abnormality such as arrhythmia that cannot be checked in the fetal heart rate diagram of the cardiotocogram.

In the maternal and fetal monitoring device of the invention, the display means may be able to display the fetal bioelectric signal in the predetermined period on the fetal bioelectric signal diagram to be distinguished from the fetal bioelectric signal in another period adjacent to the predetermined period.

It is possible to display the fetal bioelectric signal in the predetermined period of the cardiotocogram and the fetal bioelectric signal in another period in a distinguishable manner, and thus to easily check the fetal bioelectric signal in the predetermined period and the fetal bioelectric signal in the period adjacent to the predetermined period.

The maternal and fetal monitoring device of the invention may further include maternal electrocardiogram signal acquisition means configured to acquire a maternal electrocardiogram signal. The display means may be able to display a maternal electrocardiogram that displays the maternal electrocardiogram signal together with the fetal bioelectric signal diagram.

The maternal electrocardiogram is displayed together with the fetal bioelectric signal diagram. Therefore, it is possible to find a correlation between the fetal bioelectric signal and the maternal electrocardiogram signal on the same time axis.

The maternal and fetal monitoring device of the invention may further include an event input unit that records event information. The display means may be able to display the event information on the cardiotocogram together with the information on the fetal heart rate and the labor pain intensity.

Event information such as a posture change of a maternal body or a drip start to the maternal body can be input and recorded by the event input unit, and the event information can be displayed on the cardiotocogram together with the information on the fetal heart rate and the labor pain intensity. Therefore, it is possible to easily recognize the variation in the fetal heart rate or the like caused by these events and thus to easily diagnose a maternal or fetal situation.

The maternal and fetal monitoring device of the invention may further include variation detection means configured to detect variation in the fetal bioelectric signal. The display means may be able to display an occurrence point of the variation in the fetal bioelectric signal on the cardiotocogram together with the information on the fetal heart rate and the labor pain intensity.

It is possible to detect the variation in the fetal bioelectric signal based on the information stored in advance and to display the occurrence point of the variation on the cardiotocogram together with the information on the fetal heart rate and the labor pain intensity. Therefore, it becomes easier to grasp a change in the fetal heart rate and the labor pain intensity accompanying the variation in the fetal bioelectric signal. Further, it becomes easy to find the correlation among the fetal heart rate, the labor pain intensity, and the fetal bioelectric signal, and it is possible to resolve the misunderstanding in the diagnosis based on only the cardiotocogram in the related art.

In the maternal and fetal monitoring device according to the invention, the display means may be able to further display a time axis indicating the entire period during which the fetal heart rate, the labor pain intensity, and the fetal bioelectric signal are acquired and a display range frame indicating a display range of the cardiotocogram.

Normally, since the maternal and fetal monitoring is performed for several hours, determination is difficult to be made when the cardiotocogram in the entire period is displayed. Therefore, a measurement result of approximately 15 minutes is displayed on one screen. For this reason, it is possible to easily grasp at which timepoint in the entire monitoring period the displayed cardiotocogram is, by displaying the time axis and the display range frame.

Further, it is preferable that the event information display unit is able to display an icon indicating a time when the event information is input on the time axis. In this case, since the time of the displayed cardiotocogram and the event occurrence time can be easily compared, it is possible to quickly check maternal and fetal states.

A display device for monitoring device of the invention is connectable to a maternal and fetal monitoring device including fetal heart rate acquisition means configured to acquire a fetal heart rate, labor pain intensity acquisition means configured to acquire a maternal labor pain intensity, and fetal bioelectric signal acquisition means configured to acquire a fetal bioelectric signal. The display device includes display means capable of simultaneously displaying a cardiotocogram that displays the fetal heart rate and the labor pain intensity side by side on the same time axis over time as a graph and a fetal bioelectric signal diagram that displays the fetal bioelectric signal.

The display device of the invention may further include a designated period input unit capable of designating a predetermined period on the cardiotocogram. The display means may be able to display the fetal bioelectric signal in the predetermined period on the fetal bioelectric signal diagram when the predetermined period is designated by the designated period input unit.

In the display device of the invention, the maternal and fetal monitoring device may include maternal electrocardiogram signal acquisition means configured to acquire maternal electrocardiogram signal. The display means may be able to display a maternal electrocardiogram that displays the maternal electrocardiogram signal together with the fetal bioelectric signal diagram.

The display device of the invention may further include an event input unit that records event information. The display means may be able to display the event information on the cardiotocogram together with the information on the fetal heart rate and the labor pain intensity.

In the display device of the invention, the maternal and fetal monitoring device may include variation detection means configured to detect variation in the fetal bioelectric signal. The display means may be able to display an occurrence point of the variation in the fetal bioelectric signal on the cardiotocogram together with the information on the fetal heart rate and the labor pain intensity.

In the display device according to the invention, an event information display unit that displays a time axis indicating the entire period during which the maternal and fetal monitoring device acquires the fetal heart rate, the labor pain intensity, and the fetal bioelectric signal and a display range frame indicating a display range of the cardiotocogram may be able to be further displayed.

Further, the event information display unit may be able to further display an icon indicating a time when the event information is recorded on the time axis.

### Advantageous Effects of Invention

According to the invention, it is possible to easily and accurately determine the maternal and fetal situation by optimizing and displaying, together with the cardiotocogram that displays the fetal heart rate and the maternal labor pain intensity side by side over time, the information such as the fetal bioelectric signal closely related to these pieces of information.

### BRIEF DESCRIPTION OF DRAWINGS

[Fig. 1] It is a block diagram illustrating a configuration of a maternal and fetal monitoring device according to a first embodiment of the present invention.
[Fig. 2] It is a block diagram illustrating a configuration of a processing unit of the maternal and fetal monitoring device illustrated in Fig. 1.
[Fig. 3] It is a front view of a display unit of the maternal and fetal monitoring device according to the first embodiment of the present invention and is a diagram showing an example of sequentially displaying maternal and fetal information being measured.
[Fig. 4] It is a front view of the display unit of the maternal and fetal monitoring device according to the first embodiment of the present invention and is a diagram showing an example of reproducing and displaying information accumulated in a storage unit.
[Fig. 5] It is an external view of the maternal and fetal monitoring device according to the first embodiment of the present invention.
[Fig. 6] It is a diagram showing a configuration of a display device and a monitoring device to which the display device is connected according to a second embodiment of the present invention.

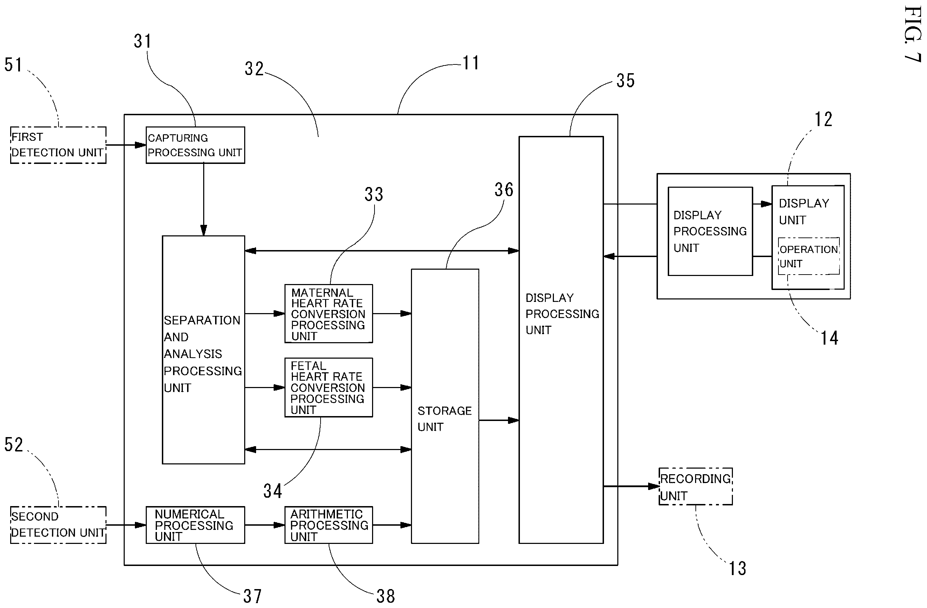

[Fig. 7] It is a diagram showing a processing process in the display device and the monitoring device shown in Fig. 6.

### DESCRIPTION OF EMBODIMENTS

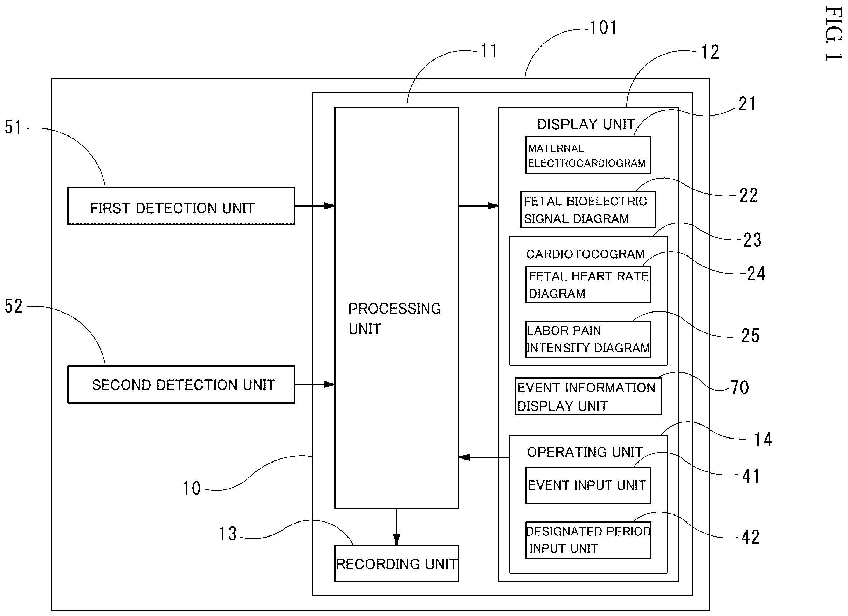

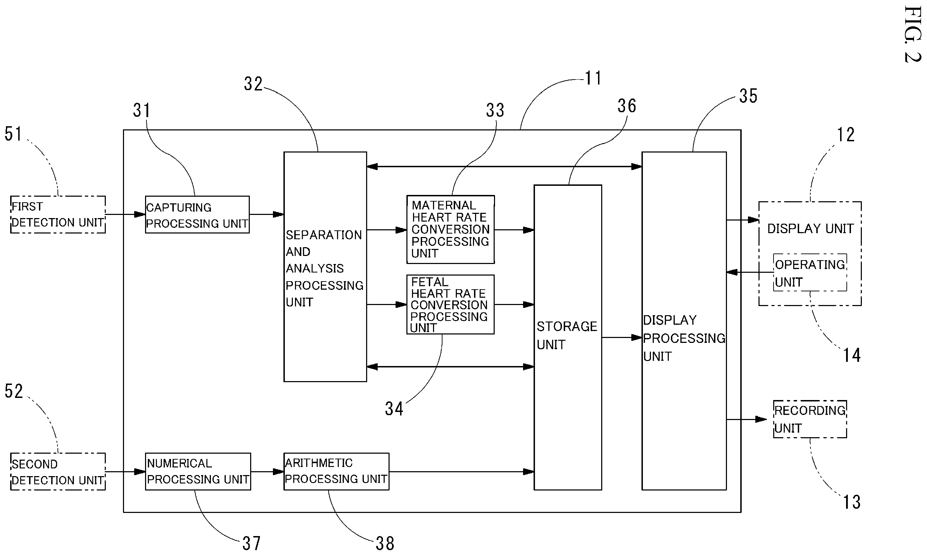

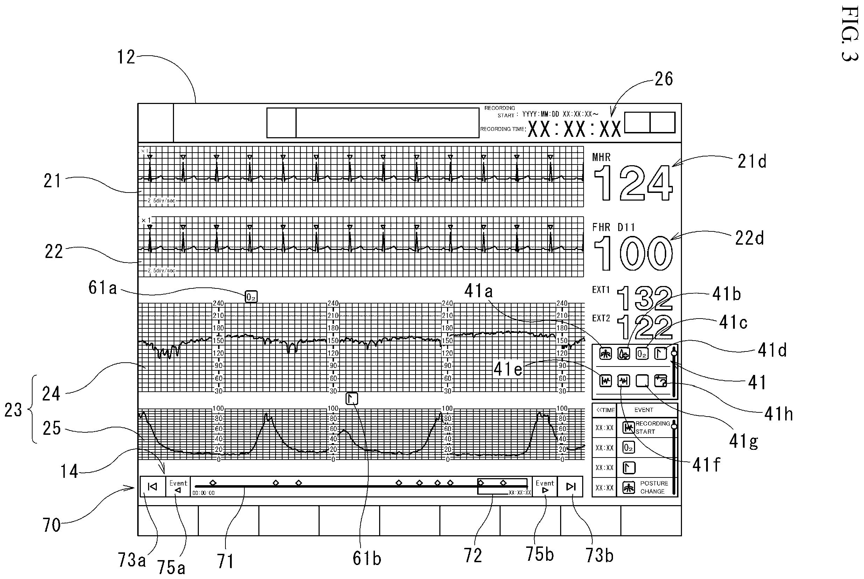

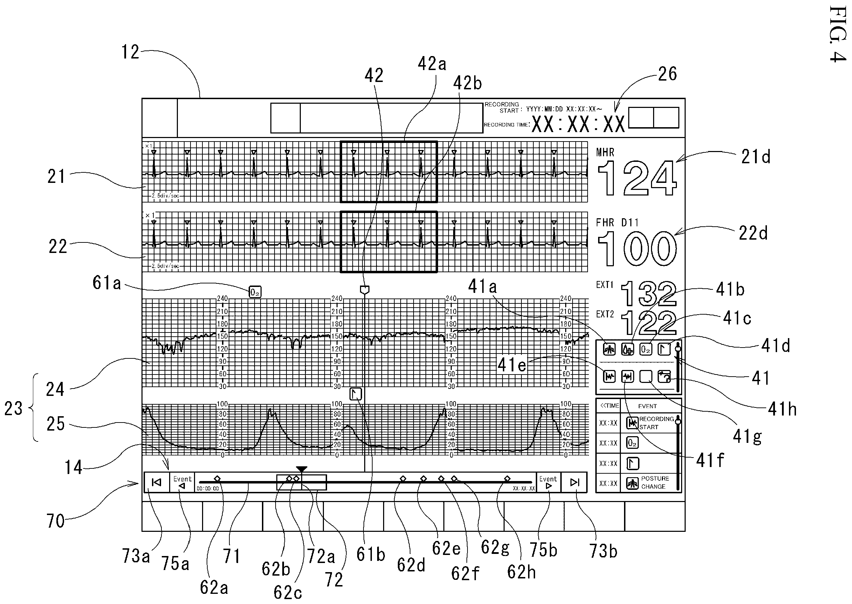

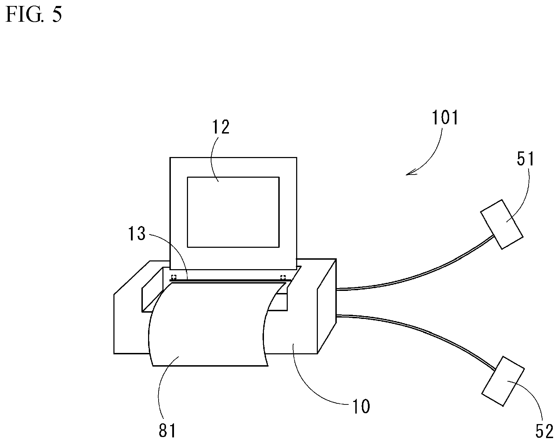

Hereinafter, each Embodiment of a maternal and fetal monitoring device and a display device for monitoring device according to the present invention will be described below with reference to drawings. Fig. 1 is a block diagram illustrating a configuration of a maternal and fetal monitoring device (hereinafter "monitoring device") 101 according to a first embodiment of the present invention. Fig. 2 is a block diagram illustrating a configuration of a processing unit 11 of the monitoring device 101. Figs. 3 and 4 are front views (screen diagrams) of a display unit 12 of the monitoring device 101. Fig. 5 is an external view of the monitoring device 101.

### [Overall Configuration of Maternal and Fetal Monitoring Device]

As shown in the entire block diagram of Fig. 1, the monitoring device 101 according to the embodiment includes a device main body 10, and a first detection unit 51 and a second detection unit 52 connected to the device main body 10.

The first detection unit 51 is a sensor for acquiring signals for detecting four pieces of information of a maternal electrocardiogram signal, a fetal bioelectric signal, a maternal heart rate, and a fetal heart rate. The second detection unit 52 is a sensor for acquiring a signal for detecting a maternal labor pain intensity. Details of the configurations of the detection units 51 and 52 and a method for detecting the maternal electrocardiogram signal, the fetal bioelectric signal, the maternal heart rate, the fetal heart rate, and the labor pain intensity will be described below.

As shown in the block diagram of Fig. 1, the device main body 10 includes the processing unit 11 including a computer that performs arithmetic processing on the signals acquired from the first detection unit 51 and the second detection unit 52, the display unit 12 (refer to Figs. 3 and 4) that displays maternal and fetal information obtained in the processing unit 11, and a recording unit 13 that outputs a fetal heart rate curve and a labor pain intensity curve of the fetal heart rate and the labor pain intensity to a recording sheet using at least pieces of information on the fetal heart rate and the labor pain intensity among the pieces of maternal and fetal information obtained in the processing unit 11.

The first detection unit 51 is composed of a plurality of abdominal electrodes, a plurality of chest electrodes, and an ultrasonic sensor that are respectively attached to the maternal body (not shown). The abdominal electrode attached to the maternal abdomen detects a biopotential signal in which various signals, such as the maternal electrocardiogram signal generated from a maternal heart, a uterine electromyogram signal, a maternal electromyogram signal, and the fetal bioelectric signal generated from a heart of the fetus in a maternal uterus, are combined. The chest electrode attached to the maternal chest detects the maternal electrocardiogram signal that does not include the fetal bioelectric signal and the like. The ultrasonic sensor attached to the maternal abdomen detects an ultrasonic signal including a fetal Doppler signal of a heartbeat cycle of the fetal heart.

As shown in the block diagram of Fig. 1, each signal detected by the first detection unit 51 is transmitted to the processing unit 11 of the device main body 10 and is used for extracting the fetal bioelectric signal in the processing unit 11. In the monitoring device 101 according to the first embodiment, an electrocardiogram signal processing method described in Patent Literature 3 (Japanese Patent No. 4590554) is employed to extract the fetal bioelectric signal.

More specifically, as shown in Fig. 2, the processing unit 11 is provided with a capturing processing unit 31, a separation and analysis processing unit 32, a maternal heart rate conversion processing unit 33, a fetal heart rate conversion processing unit 34, a display processing unit 35, and a storage unit 36. First, each signal detected by the first detection unit 51, that is, the biopotential signal detected from the abdominal electrode, the maternal electrocardiogram signal detected from the chest electrode, and the ultrasonic signal including the fetal Doppler signal detected from the ultrasonic sensor is transmitted to the capturing processing unit 31 from the first detection unit 51 and converted into data suitable for analysis in the separation and analysis processing unit 32 in the capturing processing unit 31. Specifically, signal processing such as signal amplification, AD conversion, and data segmentation for recording and storing processing is performed.

The processed biopotential signal, maternal electrocardiogram signal, and fetal Doppler signal (ultrasonic signal) are transferred from the capturing processing unit 31 to the separation and analysis processing unit 32. The separation and analysis processing unit 32 performs electrocardiogram signal processing and extracts the fetal bioelectric signal from the biopotential signal.

The fetal bioelectric signal extracted by the separation and analysis processing unit 32 is transferred from the separation and analysis processing unit 32 to the fetal heart rate conversion processing unit 34. In the fetal heart rate conversion processing unit 34, the fetal heart rate is calculated from a change in R wave intervals obtained from the fetal bioelectric signal.

The maternal electrocardiogram signal detected by the chest electrode is further transferred from the separation and analysis processing unit 32 to the maternal heart rate conversion processing unit 33. In the maternal heart rate conversion processing unit 33, the maternal heart rate is calculated from a change in R wave intervals obtained from the maternal electrocardiogram signal.

These fetal bioelectric signal, fetal heart rate, maternal electrocardiogram signal, and maternal heart rate are stored in the storage unit 36.

Further, the separation and analysis processing unit 32 has a function of detecting variation in the fetal bioelectric signal or fetal heart rate, arrhythmia, or the like based on the information stored in the storage unit 36 in advance, a function of detecting an analysis error, and the like (variation detection means). A variation occurrence point of the fetal bioelectric signal, the analysis error, or the like detected by the separation and analysis processing unit 32 is stored in the storage unit 36 and displayed at a corresponding position on the display unit 12 (on the cardiotocogram 23). The display of the variation occurrence point of the fetal bioelectric signal, the analysis error, or the like will be described in detail in the description of the display unit 12 described below.

As described above, in the monitoring device 101 according to the first embodiment, the fetal heart rate, the fetal bioelectric signal, and the maternal electrocardiogram signal are acquired by the first detection unit 51, and the capturing processing unit 31, the separation and analysis processing unit 32, and the fetal heart rate conversion processing unit 34 of the processing unit 11. Fetal heart rate acquisition means, fetal bioelectric signal acquisition means, and maternal electrocardiogram signal acquisition means according to the invention are composed of these first detection unit 51, capturing processing unit 31, separation and analysis processing unit 32, and fetal heart rate conversion processing unit 34. In the monitoring device 101 according to the embodiment, the separation and analysis processing unit 32 and the storage unit 36 detect the variation in the fetal bioelectric signal. The variation detection means according to the invention is composed of the separation and analysis processing unit 32 and the storage unit 36.

The second detection unit 52 is composed of a labor pain transducer attached to the maternal abdomen and detects the labor pain intensity signal. The labor pain transducer is a pressure-sensitive sensor that detects an increase in pressure (labor pain intensity) caused by expansion and compression of the maternal abdomen during uterine contraction. As shown in the block diagram of Fig. 1, the labor pain intensity signal detected by the second detection unit 52 is transmitted to the processing unit 11 of the device main body 10 and is converted into a signal suitable for drawing processing in the processing unit 11.

More specifically, as shown in Fig. 2, the processing unit 11 is further provided with a numerical processing unit 37 and an arithmetic processing unit 38 in addition to the display processing unit 35 and the storage unit 36 described above. The labor pain intensity signal is transmitted from the second detection unit 52 to the numerical processing unit 37, and the numerical processing unit 37 performs the signal processing such as signal amplification, AD conversion, and data segmentation for recording and storing processing. The labor pain intensity signal subjected to the signal processing by the numerical processing unit 37 is transferred to the arithmetic processing unit 38. In the arithmetic processing unit 38, the labor pain intensity corresponding to 0 to 100 is calculated. The labor pain intensity is stored in the storage unit 36.

As described above, in the monitoring device 101 according to the first embodiment, the labor pain intensity is acquired by the second detection unit 52, and the numerical processing unit 37 and the arithmetic processing unit 38 of the processing unit 11. Labor pain intensity acquisition means according to the invention is composed of the second detection unit 52, the numerical processing unit 37, and the arithmetic processing unit 38.

The maternal electrocardiogram signal, fetal bioelectric signal, maternal heart rate, fetal heart rate, and labor pain intensity stored in the storage unit 36 are transmitted to the display processing unit 35. In the display processing unit 35, the drawing processing is performed based on these signals to create a maternal electrocardiogram 21 (maternal electrocardiogram waveform) based on the maternal electrocardiogram signal, a fetal bioelectric signal diagram 22 (fetal bioelectric signal waveform) based on the fetal bioelectric signal, a fetal heart rate diagram 24 based on the fetal heart rate, and a labor pain intensity diagram 25 based on the labor pain intensity signal. As shown in the block diagram of Fig. 1 and the screen diagrams of the display unit 12 of Figs. 3 and 4, the display unit 12 displays the maternal electrocardiogram 21, the fetal bioelectric signal diagram 22, the fetal heart rate diagram 24, and the labor pain intensity diagram 25.

The information such as the variation occurrence point of the fetal bioelectric signal, the analysis error, or the like detected by the separation and analysis processing unit 32 is also subjected to the drawing processing by the display processing unit 35 and is displayed as an event icon on the cardiotocogram 23 (fetal heart rate diagram 24) together with the information on the fetal heart rate and the labor pain intensity. The cardiotocogram 23 is a graph in which the fetal heart rate (fetal heart rate diagram 24) and the labor pain intensity (labor pain intensity diagram 25) are displayed side by side on the same time axis over time.

As described above, in the monitoring device 101 according to the first embodiment, the information such as the variation occurrence point of the fetal bioelectric signal, the analysis error, or the like is displayed by the display processing unit 35 of the processing unit 11 and the display unit 12 together with the pieces of information on the maternal electrocardiogram 21, the fetal bioelectric signal diagram 22, and the cardiotocogram 23. Display means according to the invention is composed of the display processing unit 35 and the display unit 12.

The detection method in each of the detection units 51 and 52 and the processing method in the processing unit 11 used in the maternal and fetal monitoring device 101 according to the first embodiment are examples. Each method of measuring and calculating the maternal electrocardiogram signal, the fetal bioelectric signal, the fetal heart rate, and the labor pain intensity is not particularly limited.

In the first embodiment, each of the detection units 51 and 52 detects the signal by an external measurement method. However, it is also possible to use detection means by an internal measurement method or detection means by an external measurement method different from the embodiment.

Specifically, in the monitoring device 101 according to the first embodiment, the maternal heart rate and the fetal heart rate are calculated from the maternal electrocardiogram signal and the fetal bioelectric signal based on the signals detected by the external measurement method. However, the method of acquiring the maternal heart rate and the fetal heart rate is not limited thereto. For example, in the maternal and fetal monitoring device, it is possible to directly acquire (internal measurement method) the maternal heart rate and the fetal heart rate using the electrodes or the like directly attached to the maternal body and the fetus.

Next, display contents of the display unit 12 will be described more specifically. As shown in Figs. 3 and 4, the display unit 12 has a display function of displaying the maternal and fetal information and can simultaneously display the fetal bioelectric signal diagram 22 that displays the waveform of the fetal bioelectric signal and the maternal electrocardiogram 21 that displays the waveform of the maternal electrocardiogram signal together with the cardiotocogram 23 that displays the fetal heart rate (fetal heart rate diagram 24) and the labor pain intensity (labor pain intensity diagram 25) side by side on the same time axis over time as the graph.

Fig. 3 shows the display unit 12 in a state where the acquired information is sequentially displayed substantially in real time (real time mode), and Fig. 4 shows the display unit 12 in a state where the stored information is reproduced and displayed (reproduction mode). In any mode, a time range displayed on the maternal electrocardiogram 21 and the fetal bioelectric signal diagram 22 is shorter than a time range displayed on the cardiotocogram 23, which is approximately one-thousandth of the cardiotocogram 23. Therefore, a display magnification is approximately 1000 times. For example, a display width of the cardiotocogram 23 is set to ten and several minutes while the display width of the maternal electrocardiogram 21 and the fetal bioelectric signal diagram 22 is set to ten and several seconds. Accordingly, the maternal electrocardiogram waveform and the fetal bioelectric signal waveform can be displayed in more detail in the maternal electrocardiogram 21 and the fetal bioelectric signal diagram 22.

The information on the cardiotocogram 23 (fetal heart rate diagram 24 and labor pain intensity diagram 25) at the right end (the right end of the horizontal axis which is a time axis) of Figs. 3 and 4 is newer information. The display unit 12 is provided with a recording time display unit 26 that displays a recording time. The recording time display unit 26 displays a time at which information (latest information on the screen) at the right end of the cardiotocogram 23 is acquired.

In Figs.3 and 4, a fetal heart rate digital display unit 22d that digitally displays (numerically displays) the fetal heart rate is provided on a right side of the fetal bioelectric signal diagram 22, and thus it is possible to simultaneously check the fetal heart rate together with the fetal bioelectric signal waveform of the fetal bioelectric signal diagram 22 on one screen of the display unit 12. Similarly, a maternal heart rate digital display unit 21d that digitally displays (numerically displays) the maternal heart rate is provided on the right side of the maternal electrocardiogram 21, and thus it is possible to simultaneously check the maternal heart rate together with the maternal electrocardiogram signal waveform of the maternal electrocardiogram 22 on one screen of the display unit 12.

The display unit 12 can switch and display a plurality of screens in addition to the screens shown in Figs. 3 and 4.

The display unit 12 is composed of a touch panel display (monitor) that has both the display function and an input function and also functions as an operating unit 14. Therefore, the monitoring device 101 operates (presses or touches) each of operation switches (event input buttons 41a to 41d, period feed buttons 73a and 73b, event display feed buttons 75 and 75b, and the like) displayed on the display screen of the display unit 12 to perform various operations.

For example, in a lower right portion of the display unit 12 shown in Figs. 3 and 4, there is provided an event input unit 41 to which event information such as a posture change of the maternal body or a drip start to the maternal body is input together with the information on the fetal heart rate and the labor pain intensity for recording the pieces of information. The plurality of event input buttons 41a to 41d and the like are displayed on the event input unit 41. By operating these event input buttons 41a to 41d, the event information together with the information on the fetal heart rate and the labor pain intensity can be input to the storage unit 36 and recorded together with an event occurrence time.

For example, the event input buttons 41a to 41d shown in Fig. 3 are indicated by icons corresponding to each piece of event information. The event input button 41a corresponds to the posture change of the maternal body, the event input button 41b corresponds to the drip start to the maternal body, the event information input button 41c corresponds to an oxygen administration to the maternal body, and the event input button 41d corresponds to a fetal movement, respectively. With the operation of each of the event input buttons 41a to 41d corresponding to the occurrence of these events, each piece of event information is associated with the information on the fetal heart rate and the labor pain intensity at the timepoint and stored in the storage unit 36. These pieces of event information are subjected to the drawing processing in the display processing unit 35 and are displayed by event icons 61a and 61b together with the cardiotocogram 23 (fetal heart rate diagram 24) on the display unit 12 (refer to Figs. 3 and 4).

An event sign 41e corresponds to event information indicating a recording start timepoint of the cardiotocogram 23, and an event sign 41f corresponds to event information indicating a recording end time of the cardiotocogram 23. The recording of these pieces of event information (recording start timepoint and recording end timepoint) is automatically performed at the start and end of measurement of the fetal heart rate, the labor pain intensity, and the like, not by the operation of the event input button. An event sign 41g corresponds to event information indicating the variation occurrence point of the fetal bioelectric signal. When this event occurs (when the fetal bioelectric signal varies during measurement of the fetal heart rate, the labor pain intensity, and the like), the event information of the variation occurrence detected (acquired) by the separation and analysis processing unit 32 is automatically stored in the storage unit 36. Even when the analysis error occurs in the separation and analysis processing unit 32, the event information (corresponding to an event sign 41h) indicating the occurrence of the analysis error or the like is automatically stored in the storage unit 36. These automatically stored pieces of event information are subjected to the drawing processing by the display processing unit 35 and displayed by event icons (not shown) having the same design as the event signs 41e to 41h on the cardiotocogram 23 of the display unit 12.

The display unit 12 of the monitoring device 101 can display not only the maternal electrocardiogram 21, the fetal bioelectric signal diagram 22, and the cardiotocogram 23 (fetal heart rate diagram 24 and labor pain intensity diagram 25) based on the processed maternal electrocardiogram signal, fetal bioelectric signal, fetal heart rate, and labor pain intensity substantially in real time (Fig. 3) but also the maternal electrocardiogram 21, the fetal bioelectric signal diagram 22, and the cardiotocogram 23 (fetal heart rate diagram 24 and labor pain intensity diagram 25) based on the accumulated maternal electrocardiogram signal, fetal bioelectric signal, fetal heart rate, and labor pain intensity by calling the stored information already accumulated in the storage unit 36 (Fig. 4).

An event information display unit 70 is displayed at the bottom of the display unit 12, in which a scroll bar (time axis) 71 representing the entire information accumulation time from the recording start timepoint (the entire period in which the fetal heart rate, the labor pain intensity, and the fetal bioelectric signal are acquired in the monitoring device 101) and a scroll frame (display range frame) 72 indicating a display range of the cardiotocogram 23 on the scroll bar 71 are shown. That is, information within a period surrounded by the scroll frame 72 on the scroll bar 71 is displayed in the cardiotocogram 23 on the screen of the display unit 12. The recording time display unit 26 displays a time when the information at the right end of the cardiotocogram 23 is acquired.

In the state of Fig. 3 where the acquired information is displayed substantially in real time, the cardiotocogram 23 displays the latest information. Therefore, the scroll frame 72 is positioned at the right end of the scroll bar 71, and the recording time display unit 26 displays substantially a current time.

When the information is accumulated beyond a period that can be displayed on the screen (for example, 15 minutes), some pieces of information accumulated in the storage unit 36 can be displayed in the cardiotocogram 23 on the screen of the display unit 12. Information accumulated in the storage unit 36 and in a period not displayed in the cardiotocogram 23 on the screen can be displayed on the display unit 12 by moving the scroll frame 72 along the scroll bar 71 in the horizontal direction of the screen and disposing the information at a predetermined position. Fig. 4 represents a screen of the display unit 12 in the reproduction mode for displaying information at a predetermined timepoint from the accumulated information.

The scroll frame 72 can be moved to a predetermined position on the scroll bar 71 by an operation of tracing the screen (touch screen) of the display unit 12 with a finger or an operation of the period feed buttons 73a and 73b disposed on the left and right of the scroll bar 71. The display range of the cardiotocogram 23 can be changed by moving the scroll frame 72. The display range of the cardiotocogram 23 can be changed in the same manner during and after the measurement.

When the period feed buttons 73a and 73b are operated, the scroll frame 72 is moved on (before and after) a period adjacent to the display period at a current timepoint. For example, when the period feed button 73b on the right side is operated, the scroll frame 72 is moved to the right and thus the information on the fetal heart rate and the labor pain intensity in a period adjacent to the right side of the display period at the current timepoint (period after the current timepoint, information newer than the current timepoint) is displayed in the cardiotocogram 23.

When the period feed button 73a on the left side is operated, the scroll frame 72 is moved to the left side, and thus the information on the fetal heart rate and the labor pain intensity in a period adjacent to the left side of the display period at the current timepoint (period before the current timepoint, information older than the current timepoint) is displayed in the cardiotocogram 23. Further, the scroll frame 72 can be further moved to the left side by continuously operating the period feed button 73a on the left side, and thus the information on the fetal heart rate and the labor pain intensity at the position can be displayed in the cardiotocogram 23. As described above, when the period feed buttons 73a and 73b are used, the information on the fetal heart rate and the labor pain intensity for each period surrounded by the scroll frame 72 can be sequentially displayed in the cardiotocogram 23 on the screen.

As shown in Fig. 4, there is provided a designated period input unit 42 capable of designating a predetermined period (for example, several seconds) of a further short period within the display time axis (for example, ten and several minutes) displayed in the cardiotocogram 23, on the cardiotocogram 23 of the display unit 12 being reproduced. By moving the designated period input unit 42 in the horizontal direction of the screen and disposing the unit at a predetermined position within the display time axis of the cardiotocogram 23, the fetal bioelectric signal (waveform) in a period in which the designated period input unit 42 is disposed in an overlapped manner can be displayed in the fetal bioelectric signal diagram 22 and the maternal electrocardiogram signal (waveform) during the period can be displayed in a designation frame 42b of the maternal electrocardiogram 21.

The designation frame 42b is displayed in the fetal bioelectric signal diagram 22, and the fetal bioelectric signal waveform within a predetermined period designated by the designated period input unit 42 is displayed in the designation frame 42b. Therefore, the fetal bioelectric signal waveform is displayed including the period before and after (left and right) of the designation frame 42b, that is, the period before and after adjacent to the predetermined period. As described above, the fetal bioelectric signal waveform within the period corresponding to the predetermined period designated by the designated period input unit 42 is displayed within the range surrounded by the designation frame 42b, and thus the fetal bioelectric signal in the predetermined period is displayed in the fetal bioelectric signal diagram 22 so as to be distinguishable from the fetal bioelectric signal in another period.

Similar to the fetal bioelectric signal diagram 22, a designation frame 42a is also displayed in the maternal electrocardiogram 21, and the maternal electrocardiogram signal in the predetermined period and the maternal electrocardiogram signal in another period are displayed in a distinguishable manner. That is, the maternal electrocardiogram signal waveform in the predetermined period designated by the designated period input unit 42 is displayed in the designation frame 42a in the maternal electrocardiogram 21. The maternal electrocardiogram 21 and the fetal bioelectric signal diagram 22 always display information in the same time range.

The designated period input unit 42 is a vertical line-shaped marker displayed with a width of one to several pixels on the screen and is indicated by designation frames 42a and 42b in the maternal electrocardiogram 21 and the fetal bioelectric signal diagram 22 by designating a time corresponding to the width as the predetermined period. For example, when the width (one to several pixels) of the designated period input unit 42 corresponds to one second, one second is designated as the predetermined period. In the maternal electrocardiogram 21 and the fetal bioelectric signal diagram 22, when a display setting of the time axis is, for example, one-hundredth of the cardiotocogram 23 on the screen, a width 100 times the width of the designated period input unit 42 is surrounded by the designation frames 42a and 42b.

In Fig. 4, the scroll bar 71 shows the information in the entire measurement time (for example, several hours), and the cardiotocogram 23 shows the information in a time (for example, 15 minutes) surrounded by the scroll frame 72. On the contrary, the maternal electrocardiogram 21 and the fetal bioelectric signal diagram 22 show the information in a short time (for example, 10 seconds) compared with the cardiotocogram 23 in an enlarged manner, and a predetermined period (for example, one second) designated by the designated period input unit 42 is indicated by the designation frames 42a and 42b.

As means configured to display the fetal bioelectric signal in the predetermined period designated by the designated period input unit 42 and the fetal bioelectric signal in another period adjacent to the predetermined period in a distinguishable manner, it is possible to employ various means such as displaying the designation frames 42a and 42b surrounding a part of the fetal bioelectric signal diagram 22 by frame lines as shown in Figs. 3 and 4, changing a background color of the designation frames 42a and 42b to a color different from another period, changing a color of a line indicating the waveform of the fetal bioelectric signal within the predetermined period to a color different from another period, or changing a thickness of the waveform of the fetal bioelectric signal.

As described above, in the monitoring device 101 according to the first embodiment, when a predetermined period is designated by the designated period input unit 42, it is possible to enlarge and display the fetal bioelectric signal and the maternal electrocardiogram signal in the predetermined period side by side on the display unit 12 and thus to easily check the signals.

Simplified icons 62a to 62h corresponding to the event icons 61a, 61b, and the like displayed in the cardiotocogram 23 are displayed in the scroll bar 71, and thus it is possible to easily check the presence or absence of the stored event information or an occurrence point thereof in the scroll bar 71. For this reason, it is possible to easily recognize a correlation between the event information and the information on the cardiotocogram 23.

For example, in a case where a situation before and after the event occurrence corresponding to the simplified icon 62c on the scroll bar 71 is checked in the reproduction mode for checking the information accumulated in the storage unit 36 (Fig. 4), when the scroll frame 72 is moved to a position overlapping the simplified icon 62c, the waveform in the period in the scroll frame 72 is displayed in the cardiotocogram 23.

Further, when the designated period input unit 42 is moved near the event icon 61b in the cardiotocogram 23, the designation frames 42a and 42b in the maternal electrocardiogram 21 and the fetal bioelectric signal diagram 22 are moved to a position surrounding a time designated by the designated period input unit 42. Accordingly, the cardiotocogram waveform before and after the event occurrence corresponding to the event icon 61b, the maternal electrocardiogram waveform, and the fetal bioelectric signal waveform are displayed in correspondence with each other. As described above, it is possible to display side by side the cardiotocogram 23, the maternal electrocardiogram 21, and the fetal bioelectric signal diagram 22 at a predetermined timepoint and thus to compare and check each waveform.

Event display feed buttons 75a and 75b are disposed on the left and right of the scroll bar 71. It is possible to move the scroll frame 72 to a period in which event information at a position closest to the position where the scroll frame 72 at the current timepoint is disposed is recorded, by operating (pressing) these event display feed buttons 75a and 75b. It is possible to instantaneously display the information on the fetal heart rate and the labor pain intensity in the period in the cardiotocogram 23.

When the event display feed button 75b on the right side is operated, the cardiotocogram 23 is displayed with the event information (the simplified icon 62d in Fig. 4) disposed at the closest position in a right range from the scroll frame 72 at the current timepoint (position including the simplified icon 62c) as the center of the display period. When the event display feed button 75b on the right side is further operated, the cardiotocogram 23 is displayed with the event information (simplified icon 62e) on the right side of the event information (simplified icon 62d) disposed at the center position of the display period at the current timepoint as the center of the display period. As described above, in the monitoring device 101, it is possible to easily access the event information and the cardiotocogram 23 at the time of the event occurrence by operating the event display feed buttons 75a and 75b.

Further, as shown in Fig. 4, a scroll bar marker 72a indicating a time position corresponding to the designated period input unit 42 is displayed in the scroll frame 72 of the scroll bar 71. Therefore, it is possible to check where the maternal electrocardiogram 21, fetal bioelectric signal diagram 22, and cardiotocogram 23 displayed on the screen are in the entire waveform only by viewing the scroll bar 71 including the scroll frame 72 and the scroll bar marker 72a.

In the monitoring device 101, the display unit 12 is composed of the touch panel display and is used as the operating unit 14. However, the display unit 12 is not used as the operating unit 14, and a separate operating unit may be disposed at a close position adjacent to the display unit 12. In this case, since the operating unit is also disposed adjacent to the display unit 12, the operation can be performed while easily checking the display unit 12.

In the monitoring device 101, the recording unit 13 is composed of a common recorder (such as a printing device). The recording unit 13 outputs the fetal heart rate curve and the labor pain intensity curve to a recording sheet 81 (refer to Fig. 5) based on at least the pieces of information on the fetal heart rate and the labor pain intensity among the pieces of information accumulated in the storage unit 36. The cardiotocogram in which the fetal heart rate diagram and the labor pain intensity diagram are recorded (printed) side by side on the same time axis over time is created.

### [Operation of Maternal And Fetal Monitoring Device]

Next, an operation of the monitoring device 101 will be described. When the operation (measurement) of the monitoring device 101 is started, the signals acquired from the first detection unit 51 and the second detection unit 52 are processed by the processing unit 11, and the display unit 12 displays the screen with cardiotocogram 23 as shown in Fig. 3. As described above, the display unit 12 simultaneously displays the fetal bioelectric signal diagram 22 that displays the waveform of the fetal bioelectric signal and the maternal electrocardiogram 21 that displays the waveform of the maternal electrocardiogram signal together with the cardiotocogram 23. The recording unit 13 also draws the fetal heart rate curve and the labor pain intensity curve corresponding to the display unit 12 on the recording sheet 81.

In a normal measurement of the maternal electrocardiogram signal, the fetal bioelectric signal, the fetal heart rate, and the maternal heart rate, the display unit 12 displays the maternal electrocardiogram 21, the fetal bioelectric signal diagram 22, and the cardiotocogram 23 based on the maternal electrocardiogram signal, the fetal bioelectric signal, the fetal heart rate, and the labor pain intensity which are processed in real time. These pieces of information are displayed along with the time axis, and the information on the display unit 12 is sequentially updated to newly acquired information as time passes (real time mode).

As described above, the information on the cardiotocogram 23 (fetal heart rate diagram 24 and labor pain intensity diagram 25) at the right end of Fig. 3 is assumed to be the latest information. Further, the recording time display unit 26 displays a time of the measurement, which is the latest information. The time is updated with the passage of time. The pieces of information on the maternal electrocardiogram 21 and the fetal bioelectric signal diagram 22 are sequentially updated from the left end side to the right end side in Fig. 3 for each unit time. When the update point reaches the right end, the point returns to the left end and the pieces of information are updated. The maternal heart rate digital display unit 21d and the fetal heart rate digital display unit 22d numerically display the maternal heart rate and the fetal heart rate corresponding to the time at the right end in the cardiotocogram 23.

When the separation and analysis processing unit 32 detects the variation in the fetal bioelectric signal during monitoring, an icon (similar to the event icons 61a and 61b) indicating the variation occurrence point of the fetal bioelectric signal on the cardiotocogram 23 is displayed. Further, when an operator operates the event input unit 41, the event icons of the pieces of event information corresponding to the operated event input buttons 41a to 41d are displayed on the cardiotocogram 23. The pieces of event information are stored in the storage unit 36 together with the occurrence time.

In the monitoring device 101, the maternal electrocardiogram 21, the fetal bioelectric signal diagram 22, and the cardiotocogram 23 based on the maternal electrocardiogram signal, the fetal bioelectric signal, the fetal heart rate, and the labor pain intensity substantially which are processed in real time can be displayed, and, as described above, the maternal electrocardiogram 21, the fetal bioelectric signal diagram 22, and the cardiotocogram 23 based on the accumulated maternal electrocardiogram signal, fetal bioelectric signal, fetal heart rate, and labor pain intensity can be displayed and reproduced by calling the stored information already accumulated in the storage unit 36 (reproduction mode).

For example, it is possible to display the information on the fetal heart rate and the labor pain intensity in a past period on the cardiotocogram 23 of the display unit 12 by moving the scroll frame 72 on the screen of the display unit 12 shown in Fig. 4 to a position indicating the past period from the current timepoint, that is, the left side along the scroll bar 71. In this case, when the event information is recorded in the past period, the icons indicating the event information (for example, event icons 61a and 61b) are also displayed on the cardiotocogram 23.

Further, it is possible to display the waveform of the fetal bioelectric signal at a position (predetermined period) on the fetal bioelectric signal diagram 22 by moving the designated period input unit 42 displayed in an overlapped manner on the cardiotocogram 23 of the display unit 12 and disposing the unit at the predetermined position within the display time axis of the cardiotocogram 23, together with the waveform of the maternal electrocardiogram signal on the maternal electrocardiogram 21. In this case, in the maternal electrocardiogram 21 and the fetal bioelectric signal diagram 22, the fetal bioelectric signal and the maternal electrocardiogram signal in the period corresponding to the predetermined period designated on the cardiotocogram 23 are displayed so as to be distinguishable from the fetal bioelectric signal and the maternal electrocardiogram signal in another period adjacent to the predetermined period by the designation frames 42a and 42b, respectively.

As described above, in the monitoring device 101 according to the first embodiment, the display unit 12 displays the cardiotocogram 23 that displays the fetal heart rate and the labor pain intensity side by side on the same time axis over time and the fetal bioelectric signal diagram 22. Therefore, it is possible to easily check the variation in the fetal bioelectric signal within the predetermined period of the cardiotocogram 23 and to easily find abnormality such as arrhythmia that cannot be checked in the cardiotocogram 23. Further, in the monitoring device 101, the display unit 12 displays the maternal electrocardiogram 21 together with the fetal bioelectric signal diagram 22. Therefore, it is possible to find a correlation between the fetal bioelectric signal waveform and the maternal electrocardiogram signal waveform on the same time axis.

In the monitoring device 101, since the event information is displayed on the cardiotocogram 23, it is possible to easily recognize the variation in the fetal heart rate and the like caused by the event such as the posture change of the maternal body or the drip start to the maternal body and thus to easily diagnose a maternal or fetal situation.

In the monitoring device 101, since the information such as the variation occurrence point of the fetal bioelectric signal is displayed on the cardiotocogram 23, it becomes easy to grasp the change in the fetal heart rate or the labor pain intensity accompanying the variation in the fetal bioelectric signal from the cardiotocogram 23. Further, it becomes easy to find the correlation among the fetal heart rate, the labor pain intensity, and the fetal bioelectric signal from these pieces of information, and thus it is possible to resolve the misunderstanding in the diagnosis based on only the cardiotocogram 23 in the related art.

As described above, the information such as the fetal bioelectric signal closely related to these pieces of information is optimized and displayed simultaneously with the cardiotocogram 23 in the monitoring device 101. Therefore, it is possible to shorten the operation time and to easily determine the fetal situation. Therefore, it becomes easy to find the correlation among the fetal heart rate, the labor pain intensity, and the fetal bioelectric signal, and thus it is possible to resolve the misunderstanding in the diagnosis based on only the cardiotocogram 23 in the related art.

The display means including the display processing unit 35 and the display unit 12 is built in the monitoring device 101 according to the first embodiment includes. A display device for the monitoring device (hereinafter referred to as "display device") 210 according to a second embodiment of the invention can be configured in the same manner as the display means of the monitoring device 101 described above. Also in the second embodiment, the information such as the fetal bioelectric signal closely related to these pieces of information can be optimized and displayed simultaneously with the cardiotocogram that displays the fetal heart rate and the labor pain intensity side by side over time, by connecting the display device 210 to a maternal and fetal monitoring device (hereinafter "monitoring device") 201 including various means configured to detect the fetus heart rate, the fetal bioelectric signal, and the labor force intensity. Therefore, it is possible to easily determine the fetal situation.

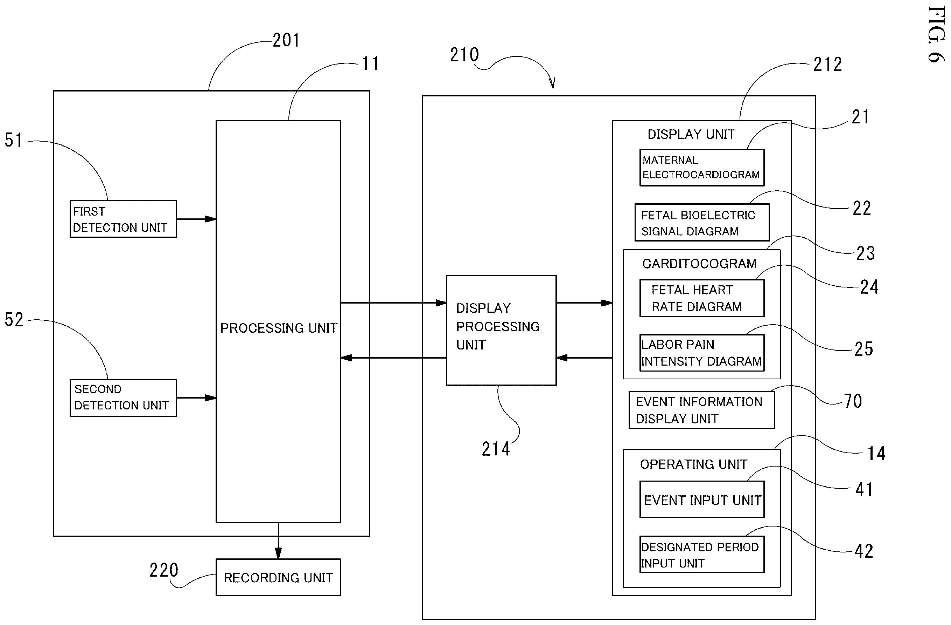

Fig. 6 shows the display device 210 according to the second embodiment of the invention. Similar to the monitoring device 101 according to the first embodiment, the monitoring device 201 according to the second embodiment to which the display device 210 is connected includes the first detection unit 51, the second detection unit 52, and the processing unit 11 (fetal heart rate acquisition means, labor pain intensity acquisition means, and fetal bioelectric signal acquisition means). However, unlike the monitoring device 101 according to the first embodiment, the display device 210 and a recording unit 220 are connected to the monitoring device 201 as separate devices.

The recording unit 220 is a printing device having the same function as that of the recording unit 13 of the monitoring device 101. The recording unit 220 includes a drawing CPU and the like and receives an input from the connected monitoring device 201 to perform printing on the recording sheet. In the monitoring device 101 described above, the recording unit may be also configured as a separate device that can be connected to the device main body 10 including the processing unit 11.

Similar to the display means (the display processing unit 35 and the display unit 12) of the monitoring device 101 described above, the display device 210 includes a display unit 212 and a display processing unit 214.

Similar to the display unit 12 described above, the display unit 212 of the display device 210 includes the fetal bioelectric signal diagram 22, the cardiotocogram 23, and the operating unit 14, and can display each piece of information and the predetermined period designated by the operating unit 14.

The display processing unit 214 of the display device 210 can display the fetal bioelectric signal diagram 22 and the cardiotocogram 23 together with the event information on the display unit 212 based on the signal input from the monitoring device 201 and transmit the information on the input event or the predetermined period in the operating unit 14 to the monitoring device 201.

In this configuration, when the monitoring device 201 includes the maternal electrocardiogram signal acquisition means (first detection unit 51 and processing unit 11) for acquiring the maternal electrocardiogram signal similar to the monitoring device 101 described above, the display unit 212 can display the maternal electrocardiogram 21 of the maternal electrocardiogram signal input from the monitoring device 201 together with the fetal bioelectric signal diagram 22.

Further, when the monitoring device 201 includes the variation detection means that detects the variation in the fetal bioelectric signal similar to the monitoring device 101 described above, the display unit 212 can display the occurrence point of the variation in the fetal bioelectric signal input from the monitoring device 201 together with the information on the fetal heart rate and the labor pain intensity on the cardiotocogram 23.

The invention is not limited to each embodiment described above, and various changes can be added within the scope not departing from the gist of the invention. For example, in the second embodiment, the configuration is employed in which the display device 210 is connected to the monitoring device 201 not having the display unit. However, a configuration may be employed in which the display device 210 according to the second embodiment is further connected to the monitoring device 101 according to the first embodiment including the display unit 12.

### Industrial Applicability

It is possible to easily determine the maternal and fetal situation by optimizing and displaying, together with the cardiotocogram that displays the fetal heart rate and the maternal labor pain intensity side by side over time, the information such as the fetal bioelectric signal closely related to these pieces of information.

### Reference Signs List

10: device main body
11: processing unit
12: display unit
13: recording unit
14: operating unit
21: maternal electrocardiogram
21d: maternal heart rate digital display unit
22: fetal bioelectric signal diagram
22d: fetal heart rate digital display unit
23: cardiotocogram
24: fetal heart rate diagram
25: labor pain intensity diagram
26: recording time display unit
31: capturing processing unit
32: separation and analysis processing unit
33: maternal heart rate conversion processing unit
34: fetal heart rate conversion processing unit
35: display processing unit
36: storage unit
37: numerical processing unit
38: arithmetic processing unit
41: event input unit
41a-41d: event input button
41e-41h: event sign
42: designated period input unit
42a, 42b: designation frame
51: first detection unit
52: second detection unit
61a, 61b: event icon
62a-62h: simplified icon (icon)
70: event information display unit
71: scroll bar (time axis)
72: scroll frame (display range frame)
72a: scroll bar marker
73a, 73b: period feed button
75a, 75b: event display feed button
81: recording sheet
101: maternal and fetal monitoring device
201: maternal and fetal monitoring device
210: display device
212: display unit
214: display processing unit
220: recording unit

## Claims

1. A maternal and fetal monitoring device comprising:
fetal heart rate acquisition means configured to acquire a fetal heart rate;
labor pain intensity acquisition means configured to acquire a maternal labor pain intensity;
fetal bioelectric signal acquisition means configured to acquire a fetal bioelectric signal; and
display means capable of simultaneously displaying a cardiotocogram that displays the fetal heart rate and the labor pain intensity side by side on the same time axis over time as a graph and a fetal bioelectric signal diagram that displays the fetal bioelectric signal.

2. The maternal and fetal monitoring device according to claim 1, further comprising:
a designated period input unit capable of designating a predetermined period on the cardiotocogram,
wherein the display means is able to display the fetal bioelectric signal in the predetermined period on the fetal bioelectric signal diagram when the predetermined period is designated by the designated period input unit.

3. The maternal and fetal monitoring device according to claim 2,
wherein the display means is able to display the fetal bioelectric signal in the predetermined period on the fetal bioelectric signal diagram to be distinguished from the fetal bioelectric signal in another period adjacent to the predetermined period.

4. The maternal and fetal monitoring device according to any one of claims 1 to 3, further comprising:
maternal electrocardiogram signal acquisition means configured to acquire a maternal electrocardiogram signal,
wherein the display means is able to display a maternal electrocardiogram that displays the maternal electrocardiogram signal together with the fetal bioelectric signal diagram.

5. The maternal and fetal monitoring device according to any one of claims 1 to 4, further comprising:
an event input unit that records event information,
wherein the display means is able to display the event information on the cardiotocogram together with the information on the fetal heart rate and the labor pain intensity.

6. The maternal and fetal monitoring device according to any one of claims 1 to 5, further comprising:
variation detection means configured to detect variation in the fetal bioelectric signal,
wherein the display means is able to display an occurrence point of the variation in the fetal bioelectric signal on the cardiotocogram together with the information on the fetal heart rate and the labor pain intensity.

7. The maternal and fetal monitoring device according to claim 5,
wherein the display means is able to further display an event information display unit that displays a time axis indicating the entire period during which the fetal heart rate, the labor pain intensity, and the fetal bioelectric signal are acquired and a display range frame indicating a display range of the cardiotocogram on the time axis.

8. The maternal and fetal monitoring device according to claim 7,
wherein the event information display unit is able to further display an icon indicating a time when the event information is input on the time axis.

9. A display device for monitoring device which is connectable to a maternal and fetal monitoring device including fetal heart rate acquisition means configured to acquire a fetal heart rate, labor pain intensity acquisition means configured to acquire a maternal labor pain intensity, and fetal bioelectric signal acquisition means configured to acquire a fetal bioelectric signal, the display device comprising:
display means capable of simultaneously displaying a cardiotocogram that displays the fetal heart rate and the labor pain intensity side by side on the same time axis over time as a graph and a fetal bioelectric signal diagram that displays the fetal bioelectric signal.

10. The display device according to claim 9, further comprising:
a designated period input unit capable of designating a predetermined period on the cardiotocogram,
wherein the display means is able to display the fetal bioelectric signal in the predetermined period on the fetal bioelectric signal diagram when the predetermined period is designated by the designated period input unit.

11. The display device according to claim 10,
wherein the display means is able to display the fetal bioelectric signal in the predetermined period on the fetal bioelectric signal diagram to be distinguished from the fetal bioelectric signal in the predetermined period and another period.

12. The display device according to any one of claims 9 to 11,
wherein the maternal and fetal monitoring device includes maternal electrocardiogram signal acquisition means configured to acquire maternal electrocardiogram signal, and
wherein the display means is able to display a maternal electrocardiogram that displays the maternal electrocardiogram signal together with the fetal bioelectric signal diagram.

13. The display device according to any one of claims 9 to 12, further comprising:
an event input unit that records event information,
wherein the display means is able to display the event information on the cardiotocogram together with the information on the fetal heart rate and the labor pain intensity.

14. The display device according to any one of claims 9 to 13,
wherein the maternal and fetal monitoring device includes variation detection means configured to detect variation in the fetal bioelectric signal, and
wherein the display means is able to display an occurrence point of the variation in the fetal bioelectric signal on the cardiotocogram together with the information on the fetal heart rate and the labor pain intensity.

15. The display device according to claim 13,
wherein an event information display unit that displays a time axis indicating the entire period during which the maternal and fetal monitoring device acquires the fetal heart rate, the labor pain intensity, and the fetal bioelectric signal and a display range frame indicating a display range of the cardiotocogram is able to be further displayed.

16. The display device according to claim 15,
wherein the event information display unit is able to further display an icon indicating a time when the event information is recorded on the time axis.
